Europäisches Patentamt

European Patent Office

Office européen des brevets

(11) Veröffentlichungsnummer: **0 290 735**

**A1**

(12) # EUROPÄISCHE PATENTANMELDUNG

(21) Anmeldenummer: **88103550.5**

(22) Anmeldetag: **08.03.88**

(51) Int. Cl.⁴: **A61F 2/36**

(30) Priorität: **15.05.87 CH 1887/87**

(43) Veröffentlichungstag der Anmeldung:
**17.11.88 Patentblatt 88/46**

(84) Benannte Vertragsstaaten:
**AT DE FR GB IT**

(71) Anmelder: **GEBRÜDER SULZER
AKTIENGESELLSCHAFT
Zürcherstrasse 9
CH-8401 Winterthur(CH)**

(72) Erfinder: **Koch, Rudolf
Oberdorfstrasse 229
CH-8267 Berlingen(CH)**
Erfinder: **Frey, Otto
Wallrütistrasse 56
CH-8400 Winterthur(CH)**

(74) Vertreter: **Dipl.-Ing. H. Marsch Dipl.-Ing. K.
Sparing Dipl.-Phys.Dr. W.H. Röhl
Patentanwälte
Rethelstrasse 123
D-4000 Düsseldorf(DE)**

(54) **Bausatz zum intraoperativen Aufbau einer Femurkopfprothese.**

(57) Der Bausatz für den Verankerungsschaft (1) einer Femurkopfprothese besteht aus einem Schaftkörper (2), einem Halsteil (8) und einem Endteil (17). Halsteil (8) und Endteil (17) sind auf den Schaftkörper (2) aufsteckbar, der dem Schaft (1) durch seine sich nahezu über die ganze Schaftlänge erstreckenden Vaterkonus (4, 5) Stabilität und Festigkeit gibt.

Der vom Schaftkörper (2) losgelöste distale Endteil (17) ermöglicht intraoperative Anpassungen des Schaftes an einen individuellen Patienten auch im distalen Bereich, ohne dass der Stabilität und Festigkeit bestimmende "Hauptteil" des Schaftes ausgetauscht weden muss.

Fig. 1

### Bausatz zum intraoperativen Aufbau einer Femurkopfprothese

Die Erfindung betrifft einen Bausatz zum intraoperativen Aufbau einer Femurkopfprothese, die aus einem den Gelenkkopf aufnehmenden proximalen Halsteil, einem Mittelteil und einem distalen Endteil zusammensetzbar ist, wobei die verschiedenen Teile über Konussteckverbindungen miteinander verbunden sind, bei denen jeweils der Vaterkonus und der Mutterkonus gleiche Konizität haben.

Um bei Femurkopfprothesen eine individuelle Anpassung gewisser geometrischer Grössen - wie z.B. die Halslänge des Prothesenhalses, die Grösse des CCD-Winkels oder Breite sowie Dicke des Schaftes in seinem, die kraftübertragende Verankerung bildenden proximalen Teil - an den einzelnen Patienten zu ermöglichen, ist es bekannt (EP-A-198 163) dem Operateur die Möglichkeit zu geben, aus einer Anzahl Halsteile auszuwählen und den ausgewählten Teil intraoperativ über eine Konussteckverbindung am proximalen Ende eines Verankerungsschaftes zu befestigen.

Eine in ihrer Zielsetzung ähnliche Konstruktion wird in der DE-A-32 05 577 beschrieben; bei dieser Prothese können Halsteile unterschiedlicher Abmessungen über Zwischenstücke auf einen Schaftteil aufgesetzt und dort fixiert werden.

Das DE-U-86 11 687 betrifft einen Bausatz für eine Schaftprothese, bei dem ein Hals, ein Mittelteil und ein distaler Endteil durch einen Zuganker miteinander verbunden werden.

Bei keiner dieser bekannten Konstruktionen ist es möglich, die Form, Länge und/oder Dicke des Schaftes im distalen Bereich zu variieren, ohne praktsich den ganzen Schaft zu ersetzen; das bedingt einen hohen zusätzlichen Aufwand für Material und Vorratshaltung, wenn eine Vielzahl von Kombinationsmöglichkeiten gegeben sein sollen.

Aufgabe der Erfindung ist, eine Bausatz für eine Femurkopfprothese zu schaffen, der eine Vielzahl von Variationsmöglichkeiten, besonders im distalen Bereich, zur individuellen Anpassung an den einzelnen Patienten bietet, ohne dass Aufwand und Platzbedarf für Herstellung und Lagerhaltung der einzelnen Teile sich erheblich vergrössern.

Erfingungsgemäss wird diese Aufgabe dadurch gelöst, dass der Mittelteil als zentraler SChaftkörper ausgebildet ist, auf dessen nach proximal und nach distal hervorstehende Vaterkonen der Halsteil und der Endteil aufsteckbar und fixierbar sind.

Der zentrale Schaftkörper, der in Verbindung mit seinen Vaterkonen sich nahezu über die ganze Schaftlänge erstreckt, verleiht der aus dem Bausatz zusammengesetzten Prothese eine hohe Festigkeit und Stabilität, insbesondere auch bei den durch die einseitige Stellung des Gelenkkopfes verursachten Biegebeanspruchungen; Festigkeit und Stabilität entsprechen daher praktisch der Festigkeit und der Stabilität einer Prothese aus einem Stück.

Durch einen an den Schaftkörper ansetzbaren, separaten distalen Endteil wird ermöglicht, mit einem oder nur wenigen Grössen für die Schaftkörper vor allem Schaftlänge und -dicke im distalen Bereich zu variieren.

Die aneinanderstossenden Trennflächen zwischen den einzelnen Teilen werden vorteilhafterweise so ausgebildet, dass sie an der Schaftoberfläche stufenlos ineinanderübergehen.

Weiterhin ist es zweckmässig, wenn der Halsteil mit einer Nase als Verdrehsicherung in eine Vertiefung des Mittelteils eingreift.

Bei einer Reihe von Schaftkonstruktionen ist der distale Bereich kreiszylindrisch ausgebildet; während wegen des unsymmetrischen Ansatzes des Prothesenhalses der Schaftquerschnitt proximal eher eine ovale ellipsenähnliche Form hat.

In neuerer Zeit werden Femurkopfprothesen sehr häufig nicht mehr distal, sondern in ihrem proximalen Bereich fixiert. Der distale Endteil hat bei solchen Prothesen die Funktion, eine Varus- oder Valgus-Stellung der Prothese im Knochen zu verhindern oder zu korrigieren. Weiterhin kann es erwünscht sein, am Femurknochen medial oder lateral die "tragenden" Belastungen zu verringern. Für beide Fälle ist es zweckmässig, wenn der distale Endteil unsymmetrisch zum Vaterkonus des Mittelteiles ausgebildet ist. Ferner kann der distale Endteil bei proximal verankerbaren Prothesen an seiner Oberfläche glatt poliert sein, um ein Einwachsen von Gewebe in diesen Endteil möglichst zu verhindern, da in seinem Bereich ein leichtes Gleiten des Schaftes im Knochen erwünscht ist.

Im folgenden wird die Erfindung anhand von Ausführungsbeispielen im Zusammenhang mit der Zeichnung näher erläutert.

Fig. 1 stellt in einer Ansicht ventral/dorsal einen Bausatz-Schaft für eine Femurkoptprothese teilweise im Schnitt dar;

Fig. 2 und 3 sind Schnitte II-II und III-III von Fig. 1;

Fig. 4 gibt gibt das distale Ende einer zweiten Ausführungsform der Erfindung wieder.

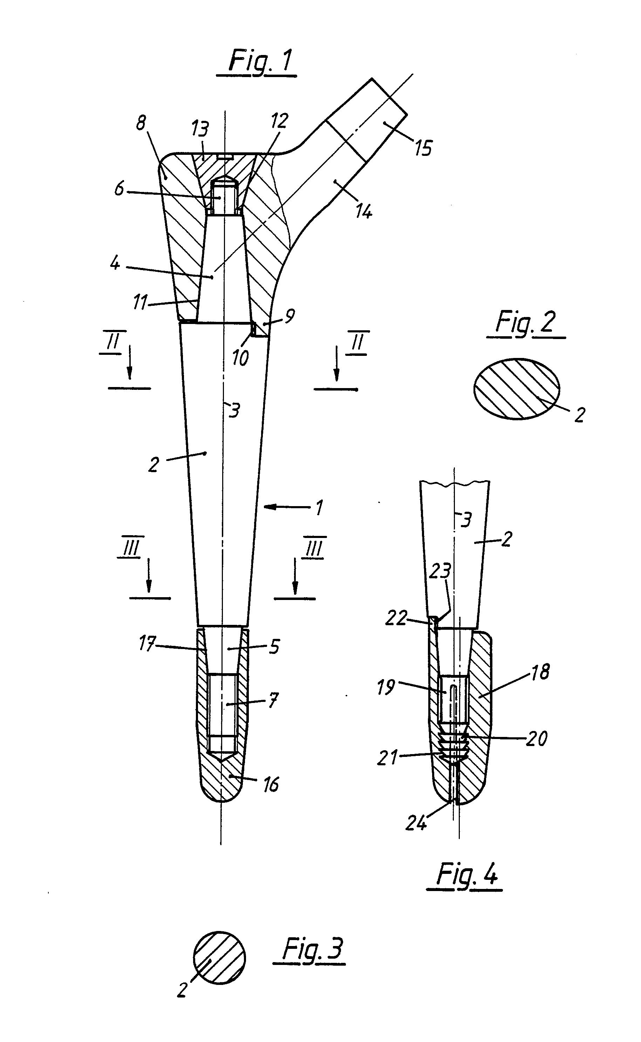

Stabilität und Festigkeit des neuen Schaftes 1 (Fig. 1) werden gewährleistet durch den als Schaftkörper 2 ausgebildeten Mittelteil; dieser besitzt in seinem proximalen Bereich einen ovalen Querschnitt, der zum distalen Ende hin in eine Kreisform übergeht. In Richtung der Schaftlängsachse 3 sind an den Schaftkörper 2 proximal und distal je ein Vaterkonus 4 und 5 angesetzt, die sich ihrerseits in Gewindebolzen 6 und 7 fortsetzen.

Auf den proximalen Vaterkonus 4 ist der Mutterkonus 11 eines Halsteiles 8 aufsteckbar, der mit einer Nase 9 in einer Ausnehmung 10 im proximalen rand des Schaftkörpers 2 eingreift, womit eine Sicherung gegen ein Verdrehen der beiden Teile 2 und 8 gegeneinander gewährleistet ist.

Der Mutterkonus 11 setzt sich nach proximal in Achsrichtung in einem Gegenkonus 12 fort, in dem ein Sicherungsbolzen 13 versenkt wird, der auf den Gewindebolzen 6 aufschraubbar ist. Der Halsteil 8 trägt den eigentlichen Prothesenhals 14, der in einem konischen Zapfen 15 zur Aufnahme des nicht gezeigten Prothesenkopfes endet.

Mit einem separaten Halsteil 8 ist dem Operateur in bekannter Weise die Möglichkeit gegeben, einen Schaftkörper 2 und einen Halsteil 8 aus einem Bausatz auf den Patienten hinsichtlich Abmessungen, Halslänge und CCD-Winkel individuell abgestimmt auszuwählen, beide Teile intraoperativ miteinander zu verbinden und anschliessend mit dem Sicherungsbolzen 13 zu sichern.

Erfindungsgemäss ist ein distalen Endteil 16 vorgesehen, der im Ausführungsbeispiel nach Fig. 1 rotationssymmetrisch zur Längsachse 3 ausgebildet ist. Er stützt sich mit einem Mutterkonus 17 auf dem Vaterkonus 5 des Schaftkörpers 2 ab und ist auf den Gewindebolzen 7 aufgeschraubt. Eine Verdrehsicherung ist bei ihn nicht vorgesehen, da dieser Endteil 16 rotationssymmetrisch zur Längsachse 3 ausgebildet ist und darüberhinaus keine grossen Torsionsbelastungen erfährt, weil er nur als im Knochen beweglicher Führungskörper dient, weshalb seine Oberfläche auch vorzugsweise hochglanzpoliert ist.

Fig. 4 zeigt eine Ausführungsform der Erfindung, bei der der Endteil 18 zur Korrektur einer Valgus-Stellung der Prothese dient. Der Endteil 18 ist daher relativ zur Längsachse 3 unsymmetrisch ausgebildet und im medialen Bereich verdickt.

Da bei einer solchen unsymmetrischen Konstruktion eine Schraubverbindung nicht mehr möglich ist, ist der an den Vaterkonus 5 des Schaftkörpers 2 anschliessende Bolzen als Aufsteckbolzen 19 mit einer Verzahnung 20 ausgebildet, in die eine Gegenverzahnung 21 des Endteils 18 eingreift. Weiterhin ist, ähnlich wie beim Halsteil 8 der ersten Ausführungsform, für die Einhaltung der richtigen "Winkelstellung" des unsymmetrischen Endteils 18 eine Nase 22 vorgesehen, die in einer Vertiefung 23 im distalen Rand des Schaftkörpers 2 sitzt.

Schliesslich ist der Endteil 18 von seinem distalen Ende her mit einem Längsschlitz 24 versehen, der mindestens nahezu mit der Längsachse 3 fluchtet.

## Ansprüche

1. Bausatz zum intraoperativen Aufbau einer Femurkopfprothese, die aus einem den Gelenkkopf aunehmenden proximalen Halsteil, einem Mittelteil und einem distalen Endteil zusammensetzbar ist, wobei die verschiedenen Teile über Konussteckverbindungen miteinander verbunden sind, bei denen jeweils der Vaterkonus und der Mutterkonus gleiche Konizität haben, dadurch gekennzeichnet, dass der Mittelteil als zentraler Schaftkörper (2) ausgebildet ist, auf dessen nach proximal und nach distal hervorstehende Vaterkonen (4, 5) der Halsteil (8) und der Endteil (17; 18) aufsteckbar und fixierbar sind.

2. Bausatz nach Anspruch 1, dadurch gekennzeichnet, das Hals-und/oder Endteil (8 bzw. 18) als Verdrehsicherung mit einer Nase (9 bzw. 22) in eine Vertiefung (10 bzw. 23) des Schaftkörpers (2) eingreifen.

3. Bausatz nach Anspruch 1, dadurch gekennzeichnet, dass der Schaftkörper (2) aus einem kreisförmigen Querschnitt an seinem distalen Ende stufenlos in einen ovalen Querschnitt an seinem proximalen Ende übergeht.

4. Bausatz nach Anspruch 1, dadurch gekennzeichnet, dass die Oberfläche des distalen Endteils (17) glatt poliert ist.

5. Bausatz nach Anspruch 1, dadurch gekennzeichnet, dass der distale Endteil (18) unsymmetrisch zum distalen Vaterkonus (5) des Schaftkörpers (2) ausgebildet ist.

_Fig. 1_

8 13 12

6 15

14

4

11

9

10

_Fig. 2_

II II

2

3

2

1

III III

17 5

7

_Fig. 4_

23

22

19 18

20

21

16

24

_Fig. 3_

2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-U-8 611 697  (CHRISTIANSEN) * Seite 7, Zeile 16 - Seite 8, Zeile 2; Figuren * --- | 1-4 | A 61 F    2/36 |
| Y | EP-A-0 099 167  (YAPP) * Seite 9, Zeilen 10,11; Figuren 1,2 * --- | 1-4 | |
| Y | US-A-3 067 740  (HABOUSH) * Spalte 2, Zeilen 61-69; Figur 3 * --- | 2 | |
| Y | FR-A-2 380 021  (RICHARDS MANUFACTURING CO., INC.) * Seite 5, Zeilen 4-10; Figuren * --- | 3 | |
| A | EP-A-0 145 641  (ANAPLIOTIS) * Seite 4, Zeile 29 - Seite 5, Zeile 8; Figuren * ----- | 1 | |

RECHERCHIERTE
SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 09-08-1988 | GLAS J. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument